# EUROPEAN PATENT SPECIFICATION

(11) **EP 2 413 831 B1**
(45) Date of publication and mention of the grant of the patent: **25.03.2015**
(21) Application number: 10759398.0
(22) Date of filing: 01.04.2010
(51) Int. Cl.: A61B 18/18, A61B 18/20, A61N 5/067, A61N 5/06

(54) **APPARATUS FOR FAT REMOVAL**
VORRICHTUNG ZUR FETTENTFERNUNG
APPAREIL DE LIPOASPIRATION

(30) Priority: 01.04.2009 US 165844 P
(43) Date of publication of application: 08.02.2012
(73) Proprietor: The General Hospital Corporation, Boston, MA 02114 (US)
(72) Inventor: MANSTEIN, Dieter, Miami, FL 33129 (US)
(74) Representative: Rupprecht, Kay
(86) International application number: PCT/US2010/029607
(87) International publication number: WO 2010/114987

(56) References cited:
- WO-A1-03/070105
- WO-A2-2008/089344
- KR-B1- 100 653 441
- KR-B1- 100 706 155
- KR-U- 20090 000 768
- KR-Y1- 200 441 715
- US-A1- 2006 058 712
- US-A1- 2007 179 481
- US-A1- 2007 264 626
- US-A1- 2008 294 150
- US-A1- 2009 069 741

## Description

### FIELD OF THE DISCLOSURE

The present disclosure relates to exemplary embodiments of an apparatus for treatment of fatty tissue, including thermal damage and/or removal of fatty tissue, by ablating holes in skin tissue that can extend down to a subcutaneous fat layer.

### BACKGROUND INFORMATION

A presence of fatty tissue in various regions of the body may be considered to be aesthetically undesirable. A reduction in the amount of fatty tissue present in various parts of the body for aesthetic reasons is becoming more common. Various procedures, both invasive and non-invasive, can be used to damage and/or remove fatty tissue directly or to facilitate its resorption by the body.

Fatty tissues can include both subcutaneous fat (which may be referred to as subdermal fat) and adipocytes (fat cells). Subcutaneous or subdermal fat can refer to fatty tissue present just below the dermis, or which may be present as small intradermal pockets of fat. Various thicknesses of such subcutaneous fatty tissue may be present in different parts of the body. For example, large amounts of subcutaneous fatty tissue can often be found in the thighs, abdomen, and upper arms. In contrast, the facial region often may have a thinner layer of fatty tissue.

Liposuction is a known invasive procedure for surgically removing a variable amount of fatty tissue from selected portions of a patient's body. Liposuction may be used, for example, to contour selected body parts such as the abdomen, buttocks, hips, thighs, etc. where larger deposits of fatty tissue may be present. Conventional liposuction can be performed by inserting a hand-held tubular instrument (e.g., a cannula) through an incision in the surface of skin tissue, such that the tip is located within or adjacent to a portion of fatty tissue to be removed. The fatty tissue can then be aspirated through the cannula, and removed from the body. A variable amount of fatty tissue may also be damaged by this procedure and not immediately removed by aspiration, but instead left within the body such that it may be reabsorbed over time.

Conventional liposuction procedures can lead to dangerous or undesirable side effects, such as disruption or severing of blood vessels, internal bleeding, pain, bruising, infection, and long recovery times. For example, conventional liposuction procedures typically include the delivery of large quantities of numbing solutions into the treatment area (tumescent analgesia). Such numbing medications (e.g. lidocaine solutions) can cause a number of side effects including, but not limited to, anaphylaxis and cardiac arrest. Liposuction procedures can often include a significant degree of movement of the cannula through the fatty tissue, which can help to mechanically break up the fatty tissue. Such motion can also disrupt or damage other tissue. For example, certain tissue surrounding the fat being removed, such as blood vessels and connective tissue, may be significantly damaged and/or partially removed along with the fatty tissue during liposuction.

Disruption and/or removal of fatty tissue can also be achieved by certain non-invasive techniques, such as physical exercise or certain nutritional supplements. However, such non-invasive techniques can have limited effectiveness and/or may require long implementation times, e.g., on the order of weeks or months, to produce noticeable results. Targeting of specific regions of fatty tissue may also not be easily achieved or even possible using these techniques.

Other non-invasive techniques which can be used for the reduction of fatty tissues may include heating of such tissue to disrupt tissue structures and promote resorption of the fatty tissue by the body. Heating of targeted fatty tissue can be performed, for example, by applying concentrated beams of light or other radiation below the skin tissue, and concentrating or focusing the beam to primarily interact with the fatty tissue while avoiding significant interaction of the beam with nearby skin and/or muscle tissue. It may also be possible to focus ultrasound waves into subcutaneous fatty tissue to heat and/or damage such tissue. However, such techniques can be potentially undesirable, as the liquefied or damaged fatty tissues remain in the body, and must be carried away naturally - otherwise, such unwanted tissues would remain in the affected areas and may cause possible infection or other undesirable effects. While it is possible that damage fatty tissue can be metabolized by the body, it may be desirable to remove at least a portion of fatty tissue that is damaged during a procedure.

Treatment of cellulite is another important clinical challenge. Cellulite is an unsightly dimpling of the skin surface that is encountered in a majority of adult women. One important factor related to the appearance of cellulite is a decrease in fibrous network that anchors the dermis to the underlying tissue. Bulging of fat near the skin surface, which can be accentuated by positioning and posture of the body, can result in a 'dimpled' appearance. There are currently no highly effective treatment options available to reduce the appearance of cellulite.

In view of the shortcomings of the above described procedures for fat damage and removal, it may be desirable to provide an apparatus that can provide damage and/or removal of fatty tissue, while reducing or avoiding at least some of the undesirable side-effects of the fat removal procedures described above.

### SUMMARY OF EXEMPLARY EMBODIMENTS OF THE DISCLOSURE

The invention is defined in the appended set of claims. Exemplary embodiments of the apparatus are provided for treatment of fatty tissue, including removal and/or thermal damage of fatty tissue. The exemplary embodiments can facilitate an ablation of portions of skin tissue to form a plurality of small holes that extend from a skin surface at least through substantially the entire thickness of the dermal layer, e.g., to a depth that reaches the subcutaneous fat layer. This exemplary procedure can result in heating, thermal damage and/or vaporization of a portion of the fatty tissue. The ablated holes can be small, e.g., less than about 1 mm in diameter, or less than about 0.5 mm in diameter, which can facilitate a rapid healing of the tissue surrounding the holes.

The tissue can be ablated using, e.g., an ablative laser such as a CO₂ laser, a mid-IR fiber laser, or the like, or another source of radiation or optical energy capable of ablating skin tissue. A control arrangement and an optical arrangement can be provided to direct electromagnetic energy from the laser onto the skin to form the plurality of holes. Such holes may be formed in a particular pattern and/or at certain separation distances from one another. For example, a 35W CO₂ laser can be used with a focal diameter, e.g., of less than about 0.5 mm, or about 0.2 mm or less. A pulse duration used to form each hole may be between, e.g., about 0.01 sec (10 msec) and about 1 sec, for example, between about 0.25 sec and about 0.5 sec. A total amount of energy directed onto skin tissue to form each hole may be between, e.g., greater than about 0.35 J (350 mJ), or greater than about 0.5 J, for example, between about 0.5 J and about 35 J, or between about 1 J and about 20 J. Such pulse energies and corresponding local fluences can be significantly larger than those used in conventional laser-based dermatological procedures.

For example, the dermal tissue in the region to be treated can be cooled and/or frozen before ablating the holes in the tissue. Such cooling or freezing can reduce the amount and/or extent of thermal damage that can occur in surrounding tissue when the holes are ablated, e.g., without significantly affecting the depth of the ablated hole formed by an energy pulse having particular properties.

According to one exemplary embodiment of the present disclosure, fatty tissue located beneath the ablated hole can be heated and/or vaporized by a portion of the energy directed to the tissue to ablate the holes. The heated fat can be thermally damaged, and then reabsorbed by the body over time. Expansion and/or vaporization of fatty tissue can cause some of the fatty tissue to be ejected from the ablated hole, which can provide an immediate reduction in the amount of local fatty tissue present.

According to another exemplary embodiment of the present disclosure, the surface of the skin can optionally be stretched before ablating the holes, which can facilitate ejection of vaporized and/or heated fatty tissue up and out of the ablated hole. Such pre-stretching can also reduce the size of the hole and/or proximal thermal damage region after the tension is released and the tissue is allowed to relax. A film or other support can be adhered to the skin surface, which can also facilitate maintaining of a passageway through the ablated hole to promote ejection of heated and/or vaporized fatty tissue. Such film can also protect the epidermis, e.g., by providing a barrier and/or thermal shield to protect the skin surface from thermal injury that could arise from heated fatty tissue that may be produced and ejected from the skin during the exemplary ablation procedures described herein.

In still another exemplary embodiment of the present disclosure, a sensor, e.g., an optical sensor, can be situated proximal to the tissue being treated. Such sensor can be configured or structured to detect a plume generated by vaporized fatty tissue during the procedure. The sensor can optionally be provided in communication with a control arrangement configured to control properties of the ablative laser or other source of optical energy. For example, the sensor and the control arrangement can be configured to detect the onset and/or occurrence of ablation of fatty tissue, prevent excessive ablation, etc.

Further, according to certain exemplary embodiments of the present disclosure, apparatus can be provided for heating subcutaneous fat. For example, it is possible to ablate a skin tissue through an entire dermal layer thereof via at least one hole. Such exemplary ablation can be performed by at least one radiation pulse provided by a radiation source. An exemplary focal diameter of the radiation pulse(s) can be less than about 0.5 mm, and have an energy greater than about 0.35 J. Further, it is possible to heat and/or vaporize fatty tissue that is provided below the dermal layer and proximal to the hole(s) using the radiation pulse(s). In addition, a control arrangement can be provided that may be configured to control at least one property of the radiation pulse, and an optical arrangement can be provided that may be configured to direct the radiation pulse onto the skin tissue

According to still another exemplary embodiment of the present disclosure, a duration of the radiation pulse can be between about 10 msec and about 1 sec, or between about 0.25 sec and about 0.5 sec. The focal diameter of the radiation pulse can be less than about 0.2 mm. An energy of the radiation pulse can be greater than about 0.5 J, or between about 0.5 J and about 35 J, or between about 1 J and about 20 J. The radiation source may comprise an ablative laser, and the ablative laser can be a CO₂ laser, a fiber laser, or the like. A plurality of holes can be ablated in a target area of skin tissue to heat and/or vaporize fatty tissue, and a distance between adjacent ones of the holes can be greater than about 1 mm, or greater than about 1.5 mm.

According to yet a further exemplary embodiment of the present disclosure, a surface of the skin tissue can be cooled and/or frozen before ablating one or more holes through the dermal layer. In addition, a stabilizing film can be adhered to a surface of the skin tissue before the ablating procedure. The stabilizing film can comprise a plastic film, a polymer film, a tape, a metallic foil, and/or a curable polymer.

In another exemplary embodiment of the present disclosure, a sensor arrangement can be provided in communication with the controller arrangement. The sensor arrangement can be configured to detect a presence of heated fat and/or vaporized fat emanating from the ablated hole(s). The controller arrangement can be configured to control a pulse energy, a pulse duration, and/or a pulse frequency, based on a signal received from the sensor arrangement. The optical arrangement can be configured to direct a plurality of pulses to a plurality of particular locations on a surface of the skin tissue. For example, the optical arrangement can control a distance between adjacent ones of the particular locations to be greater than about 1 mm, or greater than about 1.5 mm. Further. a handpiece can be provided which can include at least one portion of the optical arrangement. A plurality of pulses may also be directed onto a single location on the skin to ablate a hole to a desired depth and subsequently achieve a desired amount of heating and/or vaporization of the subcutaneous fat proximal to the hole.

These and other objects, features and advantages of the present disclosure will become apparent upon reading the following detailed description of exemplary embodiments of the present disclosure, when taken in conjunction with the appended drawings and claims.

### BRIEF DESCRIPTION OF THE DRAWINGS

Further objects, features and advantages of the present disclosure will become apparent from the following detailed description taken in conjunction with the accompanying figures showing illustrative embodiments, results and/or features of the exemplary embodiments of the present disclosure, in which:
FIG. 1A is a diagram of an exemplary apparatus that can be used to ablate holes in skin tissue and damage fatty tissue, in accordance with an exemplary embodiment of the present disclosure;
FIG. 1B is a cross-sectional diagram of an exemplary ablated hole and a portion of heated fatty tissue that is being ejected from the hole that may be effectuated by the exemplary apparatus shown in FIG. 1A;
FIG. 2 is a graph of exemplary data showing depths of ablated holes formed using various pulse durations in body-temperature, cooled, and frozen skin tissue;
FIG. 3 is a graph of exemplary data showing diameters of thermally damaged regions of skin tissue around ablated holes formed using various pulse durations in body-temperature, cooled, and frozen skin tissue;
FIG. 4A is a group of exemplary images illustrating ablated holes and corresponding regions of thermally damaged skin tissue formed by directing 70 mJ energy pulses into body-temperature, cooled, and frozen skin tissue;
FIG. 4B is a group of exemplary images illustrating ablated holes and corresponding regions of thermally damaged skin tissue formed by directing 17,500 mJ energy pulses into body-temperature, cooled, and frozen skin tissue; and
FIG. 5 is an exemplary image of regions of thermally damaged fatty tissue proximal to ablated hole.

Throughout the drawings, the same reference numerals and characters, unless otherwise stated, are used to denote like features, elements, components, or portions of the illustrated embodiments. Moreover, while the present disclosure will now be described in detail with reference to the figures, it is done so in connection with the illustrative embodiments and is not limited by the particular embodiments illustrated in the figures.

### DETAILED DESCRIPTION OF THE PREFERRED EMBODIMENTS

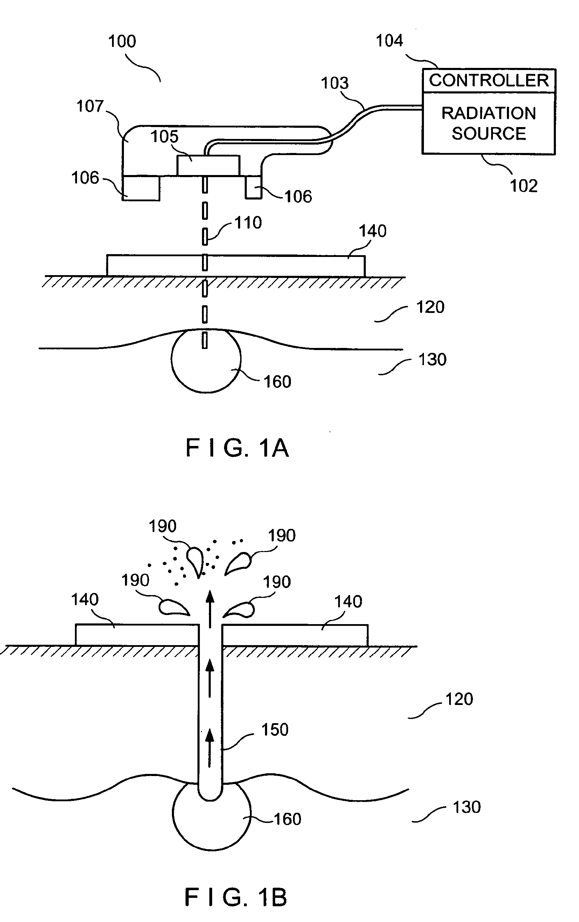

An exemplary embodiment of an apparatus 100 according to the present disclosure that can be used to heat fatty tissues is shown in Fig. 1A. The exemplary apparatus 100 can include a radiation source 102 and a control arrangement 104 that is configured to control certain properties of the radiation source 102. A handpiece 107 can be provided for directing a beam 110 of radiation produced by the radiation source 102 onto a skin tissue 120 to be treated. A sensor arrangement 106 can be provided in communication with the control arrangement 104. The sensor arrangement 106 can be connected to the handpiece 107, and/or provided proximal to the target area of the skin tissue 107 to be treated.

The apparatus 100 can also include a waveguide 103 that can be configured to direct radiation from the radiation source 102 into or through the handpiece 107. An optical arrangement 105 can also be provided to direct the radiation onto particular locations in the skin tissue 120 being treated. In certain exemplary embodiments, the radiation source 102 and/or all or a portion of the control arrangement 104 can be provided within and/or at the handpiece 107 itself.

The exemplary apparatus 100 can be structured and/or configured to direct one or more beams 110 of radiation (e.g., electromagnetic energy) onto the skin tissue 120 to ablate one or more holes therein. The skin tissue 120 can include, e.g., an upper epidermal layer and a lower dermal layer. A layer or region of fatty tissue 130 can be located below the skin tissue 120. The thickness of the layer of the skin tissue 120 can typically be between about 3 mm and about 7 mm.

The radiation source 102 can include, e.g., a laser or another source of optical radiation such as ablative electromagnetic energy, or the like. For example, the radiation source 102 can include a CO₂ laser or another type of ablative laser, e.g., a fiber laser. The radiation source 102, or a portion thereof, can be provided in the handpiece 107. Alternatively, the radiation source 102 can be provided separate from the handpiece 107, as shown in FIG. 1A.

The control arrangement 104 can be provided in communication with the radiation source 102, and configured to control and/or adjust properties of the electromagnetic energy beam(s) 110 to form the ablated holes 150, as described herein. Properties of the electromagnetic energy beam 110 which can be controlled or adjusted include, e.g., beam intensity, pulse duration, pulse rate, and/or total fluence.

The waveguide 103 can be provided to direct the radiation produced by the radiation source 102 to the handpiece 107, as shown in FIG. 1A. The waveguide 103 can include, e.g., one or more optical fibers or the like, and can preferably be flexible to facilitate multidirectional movement of the handpiece 107 relative to the radiation source 102. Other types of the waveguide(s) 103 known in the art that are suitable for directing optical radiation can also be used in various exemplary embodiments of the present disclosure.

The optical arrangement 105 can optionally be provided in the exemplary apparatus 100 to direct one or more of the beams 110 of radiation provided by the radiation source 102 onto the skin 120, e.g., to particular locations, in a particular pattern, and/or at certain separation distances on the area of the skin tissue 120 being treated. For example, the optical arrangement 105 can be provided in the handpiece 107, as shown in FIG. 1A. The optical arrangement 105 can include, for example, one or more mirrors or other reflecting surfaces, one or more prisms or other beam splitters, one or more lenses, etc. In certain exemplary embodiments of the present disclosure, the optical arrangement 105 can be configured to control or affect a focal diameter and/or focal length of the one or more beams 110, and thereby affect a resultant interaction of the beam(s) 110 with the skin tissue 120 being treated. The optical arrangement 105 can be provided in communication with the controller arrangement 104 to effect such variations in certain properties of the one or more beams 110, e.g., using conventional electromechanical actuators or the like. For example, a smaller focal diameter may generate a deeper ablated hole for a particular pulse energy by increasing the local fluence, although spreading of one or more of the beams 110 can occur deeper in the skin tissue 120, e.g., based on scattering effects.

The sensor arrangement 106 can be provided proximal to the beam 110 and/or area of the skin tissue 120 to be affected by the exemplary apparatus 100. For example, the sensor arrangement 106 may be provided on the handpiece 107, as shown in FIG. 1A. The sensor arrangement 106 can include, e.g., one or more detectors of optical radiation, such as a photodiode, a bipolar phototransistor, or a photoFET (photosensitive field-effect transistor). The sensor arrangement 107 can further include one or more reflective surfaces, lenses, or the like, and can optionally include one or more sources of low-intensity optical radiation such as a conventional LED. For example, the sensor arrangement 107 can include one or more blue LEDs. The sensor arrangement 107 can also be configured to detect interaction of the beam(s) 110 with certain types of tissue, as described in more detail below.

An exemplary ablated hole 150 that can be formed using the exemplary apparatus 100 is shown in Fig. 1B. A diameter of the ablated hole 150 can be small, e.g., less than about 1 mm in diameter, or optionally less than about 0.5 mm in diameter. Such small holes 150 can be well-tolerated by the skin tissue 120, and the small dimension can facilitate rapid healing and re-growth of the tissue surrounding the ablated hole 150. In certain exemplary embodiments of the present disclosure, the radiation source can include a single-mode fiber laser, which can facilitate ablation of such small holes.

The exemplary hole(s) 150 can extend from the surface of the skin tissue 120 at least through substantially the entire thickness of the skin tissue 120, e.g., down to the subcutaneous fat layer 130. The hole(s) 150 can also extend into the fat layer 130. The hole(s) 150 having such depth can allow a portion of the ablating energy 110 to interact with the fatty tissue 130, which can result in heating, thermal damage and/or vaporization of a target region 160 within the fatty tissue 130.

Ablating one or more holes 150 that extend into the fatty tissue 130 below the skin tissue 120 can heat and/or vaporize a particular volume of the target region 160 of the fatty tissue 130, causing expansion thereof. A portion 190 of heated fatty tissue 130 from the target region 160 can then rise up through the ablated hole 150, and exude and/or be ejected from the top of the hole 150, as shown in Fig. 1B. Such ejected or exuded fatty tissue 190 may be in a liquid and/or vapor form. This exemplary ejection of tissue can optionally be enhanced or increased, for example, by injecting the fatty tissue 130 with an aqueous solution prior to ablating the hole 150. For example, a solution containing compounds used in tumescent analgesia can be injected into the fatty tissue 130, which can facilitate a reduction of a sensation of pain that may occur during the ablation procedure. Because the water vaporizes at a lower temperature than subcutaneous fat, providing excess water in the layer of the fatty tissue 130 can promote a greater vaporization and volume expansion when the energy beam 110 interacts with the layer of the fatty tissue 130. This interaction can facilitate a further ejection of portions of the fatty tissue 190 from the hole 150, as shown in Fig. 1B.

The sensor arrangement 106 can be configured to detect the onset and/or extent of vaporization or ejection of such fatty tissue 190. For example, the ablation of the skin tissue 120, when forming one or more holes 150, can produce relatively little plume or vapor, whereas heating and/or vaporization of a portion of a volume of the target region 160 of the fatty tissue 130 can produce a dense plume from the top of the hole 150. In one exemplary embodiment of the present disclosure, the sensor arrangement 106 can include one or more photosensors arranged to detect a portion of the beam(s) 110 that can be scattered and/or reflected from the plume that includes the ejected fatty tissue 190. An increase in the amount of radiation reflected and/or scattered by the ejected fatty tissue 190 (e.g., by a plume) and detected by the sensor arrangement 106 can indicate a presence of heating and/or vaporization of the target region 160 of the fatty tissue 130. Such detection of radiation can be used to indicate that the hole 150 has penetrated the skin tissue 120 and reached the target region 160 of the fatty tissue 130.

The sensor arrangement 106 can be provided in communication with the controller arrangement 104. For example, such exemplary configuration can be used to determine and/or control a duration of heating or vaporization of the target region 160 of the fatty tissue 130 associated with a particular hole 150. The intensity of the detected radiation can also indicate the extent of vaporization or ejection of fatty tissue 190 from the ablated hole 150.

For example, a higher intensity of reflected or scattered radiation can indicate a larger degree of vaporization or ejection of fatty tissue 190. Signals provided by the sensor arrangement 106 can be used to detect the onset of penetration of the beam(s) 110 into the target region 160 of the fatty tissue 130. Such signals can also be used to limit the total energy provided to heat and/or vaporize the target region 160 of the fatty tissue 130 at one or more particular locations, which can facilitate a safer operation of the exemplary apparatus 100, e.g., by preventing excessive ablation or unwanted tissue damage.

In further exemplary embodiments of the present disclosure, the sensor arrangement 106 can include one or more photodetectors and one or more photosources of low-intensity optical radiation, such as LEDs or the like. The photodetector(s) can be configured to receive a portion of the radiation produced by the photosource(s). The photosource(s) and photodetector(s) can be arranged such that a portion of the plume of fatty tissue 190 that can be formed as described herein passes through the optical path between the photosource(s) and photodetector(s). Such exemplary configuration of the sensor arrangement 106 can provide a reduced detection of the particular radiation produced by the photosource and received by the photodetector(s) when a portion of the heated or vaporized fatty tissue is ejected from the hole(s) 150 and passes between them. The intensity of the detected particular radiation can be used to control certain properties of the beam(s) 110 of radiation provided by the radiation source 102, e.g., to facilitate a more precise control of the procedure and/or act as a safety control, as described above.

In further exemplary embodiments of the present disclosure, the sensor arrangement 106 can include one or more photosensors configured to detect reflected/scattered radiation from the beam(s) 110 and radiation provided by one or more provided low-energy photosources. The detected intensity of both types of radiation can vary with a presence and density of ejected fatty tissue 190 as described above, and signals based on such detection can be provided to the controller arrangement 104 and used to better control the fat heating/vaporization process described herein.

The thickness of the dermal layer 120 can vary significantly at different anatomical sites. Such thickness can be, e.g., between a few hundred micrometers (for example, in the eye lids) up to about half a centimeter (e.g., in the posterior region of the neck). The sensor arrangement 106 and exemplary sensing methods and apparatus described above can facilitate a determination of when the subcutaneous fatty tissue layer is reached by a radiation beam 110 during an ablative procedure such as the exemplary procedures described herein.

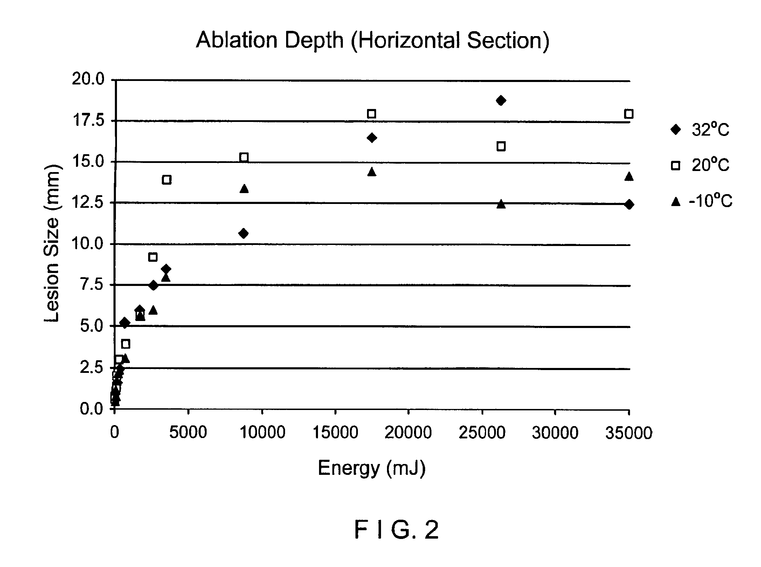

Fig. 2 shows a graph of exemplary data (e.g., lesion size vs. energy) for the ablation of the hole(s) 150 in the skin tissue 120 in accordance with certain exemplary embodiments of the present disclosure. For example, energy from a 35-watt (35 W) CO₂ laser was directed onto excised abdominal skin using a focal diameter of 0.2 mm. The skin tissue 120 was initially provided at three different temperatures, e.g., 32 °C (close to normal body temperature), 20 °C (cooled tissue), and -10 °C (frozen tissue). The thickness of the dermal layer 120 in the skin tissue 120 was approximately 8 mm. Energies greater than approximately 20 J in this data may be disregarded because the corresponding ablation depth would tend to exceed the thickness of the samples used.

The exemplary data in Fig. 2 indicate that pulse energies of about 0.35 J (350 mJ) or greater can be sufficient to form the hole(s) 150 that extended through the dermal layer of the skin tissue 120 (e.g., at least about 3 mm deep) to reach and/or penetrate into the layer of the fatty tissue 130. Pulses having higher energies can also be used to generate more thermal damage and/or vaporization of the fatty tissue 130, as described herein. For example, pulse energies can be between about 0.5 J and about 35 J, or between about 1 J and about 20 J. These exemplary energy values can correspond to a focus diameter of about 0.2 mm. Providing a smaller focus diameter for the energy beam(s) 110 can generate the ablated hole(s) 150 that penetrate to the underlying fatty layer of the fatty tissue 130 with somewhat lower pulse energies. Alternatively, larger focus diameters and higher energies may also be used in certain applications, e.g., in the buttocks and thighs where the layer of the fatty tissue 130 may be thicker than in other parts of the body.

A plurality of pulses of radiation can also be directed onto a particular location on the surface of the skin tissue 120 to ablate a hole 150 therethrough and heat or vaporize subcutaneous fat 130 located below the ablated skin tissue. The duration and/or energy of each pulse can be smaller than the exemplary values described above. The total energy of a plurality of pulses directed onto a particular location on the surface of the skin tissue 120 can be preferably within the ranges described above. For example, a total amount of energy of a plurality of pulses directed onto a particular location on the skin tissue 120 to form the hole 150 can be, e.g., greater than about 0.35 J (350 mJ), or greater than about 0.5 J, or between about 0.5 J and about 35 J, or between about 1 J and about 20 J.

A total duration of a sequence or stream of such radiation pulses can be less than about 1 sec, or less than about 0.5 sec. Such relatively short durations can be preferable to facilitate application of the plurality of pulses onto a single location on the surface of the skin tissue 120. For example, a longer duration of pulses may allow movement of the beam relative to the particular location during the ablation procedure, which can create an undesirable larger hole (e.g., greater than about 0.5 mm in diameter) that can lead to unsightly scarring and/or excessive thermal damage to the skin tissue 120. In contrast, directing a single pulse or a series of pulses having a short duration onto a particular location on the skin surface can facilitate formation of the small holes described herein.

The exemplary data shown in Fig. 2 also suggests that an increase in pulse energy beyond about 10 J did not substantially increase the observed depth of the hole(s) 150 formed. These higher pulse energies can provide additional energy within the fatty tissue 130 without generating significantly deeper ablated hole(s) 150.

A smaller beam diameter can achieve ablation of a hole through the dermal layer 120 and down to the subcutaneous fatty tissue 130 with a smaller amount of energy. The energy used to ablate skin tissue using an ablative laser is approximately 1.75 kJ per cm³ of skin tissue (e.g., a specific heat of ablation of skin), as described, e.g., in Walsh J.T. Jr and Deutsch TF, Er:YAG Laser Ablation Of Tissue: Measurement Of Ablation Rates, Lasers Surg Med. 9(4), pp. 327-37 (1989). The fluence of a particular pulse or beam 110 can be calculated as the pulse or beam energy divided by the cross-sectional beam area, which may be approximately circular in shape. The resultant depth of ablation in the skin tissue 120 can be estimated as this fluence divided by the heat of ablation of skin tissue (e.g., about 1.75 kJ per cm³). Such approximate calculations can be used to estimate and relate parameters associated with the ablative procedures provided herein, e.g., the total amount of energy provided by the radiation beam 110 (which may include one or more pulses of radiation) to ablate the hole 150 in the skin tissue 120, the area of the beam 110, and the ablation depth for reaching the subcutaneous fat layer 130 (e.g., the local thickness of the dermal layer 120). Energies greater than the beam energy estimated for ablating the hole 150 through the dermal layer 120 and into the subcutaneous fatty layer 130 can be provided for further heating and/or vaporization of fatty tissue in the target region 160 proximal to the bottom of the hole 150.

The skin tissue 120 being treated can optionally be cooled or frozen prior to applying the ablative energy beam(s) 110. Such cooling or freezing can reduce or eliminate a sensation of pain when ablating the hole(s) 150. For example, the exemplary data shown in Fig. 2 can indicate that freezing of the skin tissue 120 to a temperature of about -10 °C did not significantly decrease the depth of the ablated hole(s) 150 formed when using a pulse of the energy beam(s) 110 having a particular duration.

The latent heat for melting of frozen skin tissue is approximately 0.3 kJ/cm³. This exemplary value indicates that the total amount of energy used to melt a particular volume of skin tissue is less than about 20% of the energy required to ablate the same volume of skin tissue. This exemplary relative magnitude of energies is consistent with the observation described above that the ablation depth appears to be only minimally affected by a temperature change of the skin tissue, including freezing of the skin tissue.

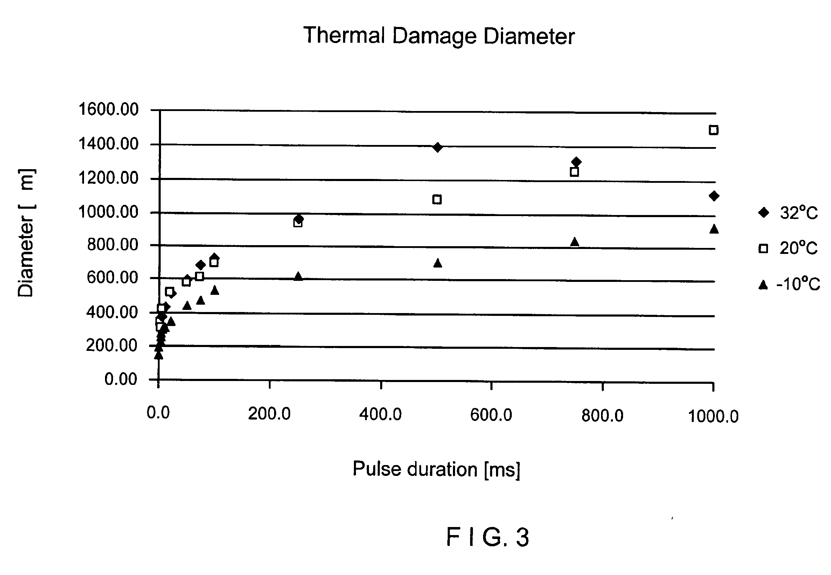

Exemplary data are shown in the graph of Fig. 3 for a diameter of a thermally damaged zone of dermal skin tissue 120 surrounding ablated holes 150 that were formed under various conditions. These exemplary data indicate that there is a regular increase in the size of the thermal damage zone with increasing pulse duration (and corresponding increase in pulse energy) up to a pulse duration of about 500 msec (i.e., 0.5 sec). Higher pulse durations and energies did not significantly increase the size of the thermal damage zone around each of the holes 150. For the exemplary data shown in Figure 3, the total energy of a pulse generated by the 35 W laser can be expressed in joules (J) as approximately 35 times the pulse duration in seconds. Alternatively, the total pulse energy in mJ is equal to about 35 times the pulse duration in msec.

The data shown in Fig. 3 also indicates that cooling or freezing of the skin tissue 120 can reduce the amount of thermally damaged dermal tissue 120 around an ablated hole 150. For example, the diameter of thermally damaged tissue that was initially frozen can be about 1/2 to 2/3 of the corresponding diameter of damaged tissue formed in body-temperature skin tissue 120. The volume ratio of the damaged tissue can be approximated as the square of the diameters of the damaged regions, which may be substantially cylindrical in shape. Accordingly, the volume of the skin (dermal) tissue 120 around the ablated hole(s) 150 that is thermally damaged in frozen skin can be, e.g., between about 1/4 to 1/2 of the damaged tissue volume formed in the skin tissue 120 that was initially at normal body temperature.

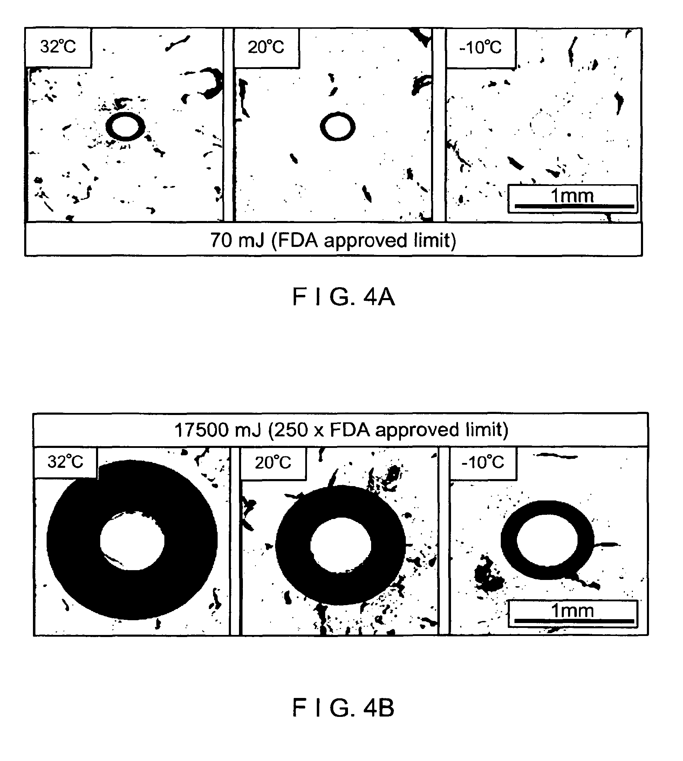

Fig. 4A shows exemplary images of thermal damage zones generated in the skin tissue 120 around the ablated holes 150 that were formed using energy pulses of about 70 mJ. The solid rings indicate the approximate size of the thermal damage zones as observed by NBTC staining of frozen sections of the skin tissue 120. The size of the holes 150 formed appear to be relatively unaffected by tissue cooling. The extent of the thermally damaged region was observed to decrease somewhat with decreasing temperature of the skin tissue 120. There was very little thermally damaged tissue observed around the holes 150 formed in frozen tissue 120 (e.g., at a temperature of-10 °C).

Fig. 4B shows further exemplary images of thermal damage zones generated in the skin tissue 120 around the ablated holes 150 that were formed using energy pulses of 17,500 mJ (e.g., 17.5 J, corresponding to a pulse duration of about 0.5 sec from the 35W CO₂ laser). The size of the holes 150 formed again appear to be relatively unaffected by tissue cooling, and are significantly larger than the holes 150 shown in Fig. 4A, which were formed using a pulse energy of about 70 mJ. The extent of the thermally damaged region was observed to decrease significantly with decreasing temperature of the skin tissue 120. There was relatively little thermally damaged tissue observed around the holes 150 formed in the frozen tissue 120, which was initially at a temperature of -10 °C.

The exemplary data and images shown in Figs. 3, 4A and 4B indicate that the amount of thermal damage generated when ablating the holes 150 in the skin tissue 120 can be significantly decreased by freezing the skin tissue 120 prior to the exemplary ablation procedure. This effect can result in part from the excess enthalpy that is needed to melt frozen tissue. For example, the amount of energy used to melt one gram of ice (e.g., substantially isothermally at 0 °C) is about the same as the energy used to heat the resulting water from 0 °C to 80 °C. The effective heat capacity of the frozen tissue can thus be much greater than that of unfrozen tissue, and a significant amount of heat generated during the ablation procedure can be absorbed by frozen tissue with a corresponding smaller temperature rise.

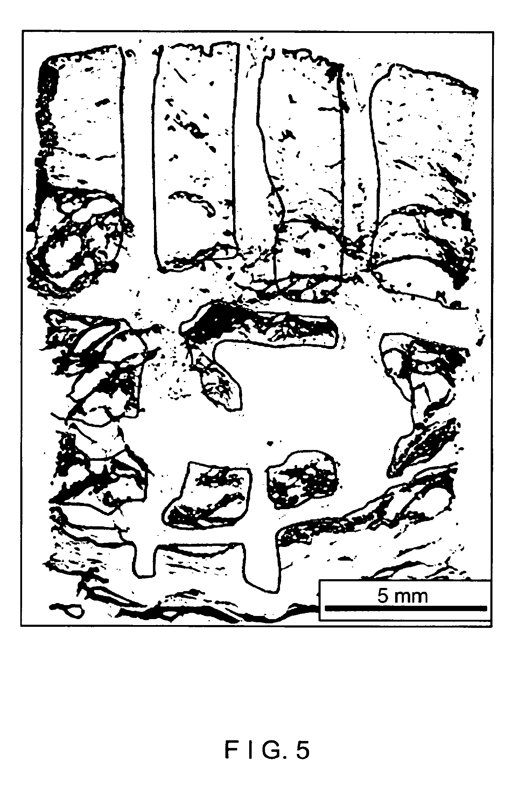

An exemplary image of the fatty tissue that was thermally damaged when forming the ablated hole 150, as described herein, is shown in Fig. 5. The portions of the thermally damaged skin tissue 120 and the fatty tissue 130 are outlined in this exemplary image. The observed damage pattern indicates that thermal damage in an upper portion of the skin layer 120 can be limited substantially to the ablated holes 150, which appear as columns in this cross-sectional image of Fig. 5. This can arise from the relatively strong structure of the skin layer 120, which can typically contain a significant amount of collagen and other connective tissues. Accordingly, the surrounding tissue in the skin (dermal) layer 120 can remain relatively intact around the ablated holes 150 that are formed therethrough.

The thermally damaged regions are more widespread in the (lower) fatty layer 130 shown in Fig. 5. Such damage can be more widespread in the fatty layer 130 based on various factors such as, e.g., spreading of heat based on melting and/or vaporization of fatty tissue, pressure-driven movement of heated fatty tissue 130 after it interacts with the energy beam(s) 110, a lesser amount of connective tissue in the fatty layer 130 (as compared to the skin layer 120) that would tend to maintain integrity of the target region 160 of the damaged fatty tissue 130, etc. These exemplary damage patterns shown in Fig. 5 indicate that using higher pulse energies can enhance the amount of damage generated in the heated volume of the target region 160 in the fatty tissue 130. Some of this thermally damaged target region 160 of the fatty tissue 130 that does not become ejected fat 190 can eventually be reabsorbed by the body, leading to a further reduction in the amount of the fat tissue 130 present after forming the ablated hole(s) 150.

The exemplary apparatus and methods described herein can also result in some tissue tightening after the ablated holes 150 are formed. Thermal damage to connective tissue (e.g., collagen) and other tissues can lead to some necrosis and contraction, e.g., after the damaged tissue heals. For example, the apparatus and methods described herein can be used to treat cellulite. Thermal damage of the fatty layer 130 as described herein can generate tissue necrosis and induce fibrosis, which may lead to additional anchoring of the overlying dermis to deeper layers in addition to disrupting some existing anchoring structures and reducing a local amount of fatty tissue. Such fibrosis can be formed as a network of fibrosis, which may result in an increased anchoring of the dermis with the underlying fatty tissue and a smoother appearance of the cellulite. Ablating between about 1 and 10 holes 150 per cm² of skin tissue, e.g., using the exemplary methods and apparatus described herein, can reduce the puckered appearance of cellulite in the treated area.

The surface of the skin tissue 120 can also be stretched before directing the electromagnetic energy 110 onto the skin tissue 120 to form the ablated hole(s) 150. For example, the skin surface can be placed in a state of tension. This exemplary procedure can assist in maintaining an open passageway through the upper portion of the ablated hole(s) 150, which can facilitate escape of the heated fatty tissue 190. In addition, the effective size of the hole(s) 150 and surrounding thermal damage area can be smaller after such tension is released, and the skin tissue 120 is allowed to contract after the ablated holes 150 are formed therein. This contraction can facilitate more rapid healing of the damaged tissue, and can assist in a reduction of a visible scarring of the skin surface after the exemplary procedure is performed.

In certain exemplary embodiments of the present disclosure, a film 140 can be provided on the surface of the skin tissue 120 prior to forming the ablated holes 150 therein. The film 140 can be configured or structured to adhere to at least a portion of the skin surface proximal to the location of the hole(s) 150 to be ablated. Accordingly, the film 140 can also assist in maintaining an open passageway at the upper portion of the ablated hole(s) 150, which can also facilitate escape of the heated fatty tissue 190 through the top of the ablated hole(s) 150. The skin tissue 120 can optionally be stretched, e.g., uni-directionally or bi-directionally, before applying the film 140 to the surface thereof, which can maintain the skin surface in a stretched state while the ablative holes 150 are formed.

The film 140 can include, e.g., a polymer or plastic film, a medical tape or other form of tape, or the like. The film 140 can also be a metallic layer or foil, e.g., a silver or aluminum foil, which can be adhered to the skin surface. Such metallic film can facilitate cooling of the underlying tissue because of a high thermal conductivity. The film 140 can be provided with an adhesive substance on one side, or an external adhesive (e.g., a surgical spray adhesive or the like) can be applied to the film 140 and/or the skin surface before applying the film 140 to the skin surface. The film 140 can also be a material that is sprayed or applied onto the skin in a liquid or gel form and allowed to dry or cure such as, e.g., Dermabond®, poly(methyl methacrylate) (PMMA), or another polymer. The film 140 can be formed from a material that can be easily ablated or vaporized, e.g., so the ablated holes 150 can be formed directly through the film 140. In further embodiments, the film 140 can be provided with a plurality of holes, such that the beam 110 can be directed through such holes and into the skin tissue 120.

As described herein, the hole(s) 150 can be ablated in a region of the skin tissue 120 to be treated. This exemplary ablation can facilitate a greater amount of fatty tissue 130 to be exuded and/or ejected from the ablated holes 150 and/or be thermally damaged and eventually reabsorbed by the body. The ablated holes 150 can preferably be spaced sufficiently far apart to maintain some healthy, undamaged tissue between the holes 150 to promote healing and re-growth of the skin tissue 120 in and around the ablated holes 150.

For example, the diameter of the thermally damaged region formed in the frozen tissue (initially at a temperature of-10 °C) using an energy pulse of 17,500 mJ can be about 0.7 mm, as shown in Fig. 4B. Accordingly, the distance between centers of the adjacent ablated holes 150 formed under such conditions can be greater than about 1 mm, or greater than about 1.5 mm, to provide a region of the undamaged skin tissue 120 between the adjacent holes 150. Appropriate separation distances can be determined for ablated holes 150 formed under various conditions in a similar manner. Larger separation distances between the adjacent ablated holes 150 can also be used. The spacing and pattern of such holes 150 can be selected based at least in part on the amount of the target regions 160 of the fatty tissue 130 to be damaged and/or ejected from the holes 150 as described herein. The spacing of the holes 150 and the total energy applied to ablate each hole 150 and subsequently heat and/or vaporize fatty tissue therethrough can both be selected to achieve a particular amount of the vaporized and/or damaged fatty tissue 130 within the target regions 160 per unit area of the skin tissue 120 treated.

The holes 150 can be formed in various patterns including, e.g., a regular square or rectangular pattern, a triangular pattern, or a random pattern. The exemplary apparatus 100 shown in Fig. 1 can be configured to form a plurality of such ablated holes 150 at appropriate separation distances and patterns as described herein. The exemplary ablation procedure can also be repeated over a particular region of the skin tissue 120, e.g., after the skin tissue 120 has been allowed to heal following an initial ablative procedure. Such multiple treatments can be used to damage and/or remove a larger volume of the fatty tissue 130 from beneath a particular area of the skin tissue 120.

## Claims

1. An apparatus for heating subcutaneous fat (130), comprising:
a radiation source arrangement (102) configured to provide at least one pulse of ablative radiation;
a control arrangement (104) configured to control at least one property of the at least one radiation pulse;
an optical arrangement (105) configured to direct the at least one radiation pulse onto a skin tissue; and
a sensor arrangement (106) provided in communication with the control arrangement (104),
wherein the at least one radiation pulse has an energy greater than0.35 J and a focal diameter that is less than 0.5 mm,
wherein at least one of the control arrangement (104) the optical
arrangement (105) is configured to at least one of control or direct the at least one radiation pulse to ablate at least one hole (150) through an entire dermal layer of the skin tissue (120), and
wherein the sensor arrangement (106) is configured to detect a presence of at least one of heated fat emanating from the at least one ablated hole (150) or vaporized fat emanating from the at least one ablated hole.

2. The apparatus of claim 1, wherein the control arrangement (104) is adapted to control a duration of the at least one radiation pulse to be between 10 msec and 1 sec.

3. The apparatus of claim 1, wherein the control arrangement (104) is adapted to control a duration of the at least one radiation pulse to be between 0.25 sec and 0.5 sec.

4. The apparatus of claim 2, wherein the control arrangement (104) is adapted to control a focal diameter of the at least one radiation pulse to be less than 0.2 mm.

5. The apparatus of claim 2, wherein the control arrangement (104) is adapted to control a total energy of the at least one radiation pulse to be greater than 0.5 J.

6. The apparatus of claim 2, wherein the control arrangement (104) is adapted to control a total energy of the at least one radiation pulse to be between 0.5 J and 35 J.

7. The apparatus of claim 2, wherein the control arrangement (104) is adapted to control a total energy of the at least one radiation pulse to be between 1 J and 20 J.

8. The apparatus of claim 2, wherein the radiation source arrangement (102) comprises an ablative laser.

9. The apparatus of claim 8, wherein the ablative laser is at least one of a CO₂ laser or a fiber laser.

10. The apparatus of claim 9, wherein the control arrangement (104) is adapted to control at least one of a pulse energy, a pulse duration, or a pulse frequency, based on a signal received from the sensor arrangement (106).

11. The apparatus of any of claims 1-9, wherein the optical arrangement (105) is adapted to direct a plurality of pulses to a plurality of particular locations on a surface of the skin tissue.

12. The apparatus of claim 11, wherein the optical arrangement (105) is adapted to control a distance between adjacent ones of the particular locations to be greater than 1 mm.

13. The apparatus of claim 11, wherein the optical arrangement (105) is adapted to control a distance between adjacent ones of the particular locations to be greater than 1.5 mm.

14. The apparatus of claim 12, further comprising a handpiece (107) which includes at least one portion of the optical arrangement (105).

## Patentansprüche

1. Vorrichtung zum Erhitzen von Unterhautfettgewebe (130), umfassend:
eine Strahlungsquellenanordnung (102), die so gestaltet ist, dass mindestens ein abladierender Strahlungspuls bereitgestellt wird;
eine Steuereinrichtung (104), die so gestaltet ist, dass mindestens eine Eigenschaft des mindestens einen Strahlungspulses gesteuert wird;
eine optische Einrichtung (105), die so gestaltet ist, dass der mindestens eine Strahlungspuls auf ein Hautgewebe gerichtet wird; und
eine Sensoranordnung (106), die in Verbindung mit der Steuereinrichtung (104) vorgesehen ist;
wobei der mindestens eine Strahlungspuls eine Energie, die größer als 0,35 J ist und einen Brennpunktdurchmesser besitzt, der kleiner als 0,5 mm ist;
wobei die Steuereinrichtung (104) und/oder die optische Einrichtung (105) so gestaltet ist, dass der mindestens eine Strahlungspuls gesteuert und/oder gelenkt wird, um mindestens ein Loch (150) durch eine unversehrte Hautschicht des Hautgewebes (120) zu abladieren; und
wobei die Sensoranordnung (106) so gestaltet ist, dass das Vorhandensein von erhitztem Fett, das aus dem mindestens einen abladierten Loch (150) herrührt, und/oder von verdampftem Fett, das aus dem mindestens einen abladierten Loch herrührt, detektiert wird.

2. Vorrichtung nach Anspruch 1, wobei die Steuereinrichtung (104) die Dauer des mindestens einen Strahlungspulses so steuern soll, dass er zwischen 10 ms und 1 s liegt.

3. Vorrichtung nach Anspruch 1, wobei die Steuereinrichtung (104) die Dauer des mindestens einen Strahlungspulses so steuern soll, dass er zwischen 0,25 s und 0,5 s liegt.

4. Vorrichtung nach Anspruch 2, wobei die Steuereinrichtung (104) einen Brennpunktdurchmesser des mindestens einen Strahlungspulses so steuern soll, dass er kleiner als 0,2 mm ist.

5. Vorrichtung nach Anspruch 2, wobei die Steuereinrichtung (104) eine Gesamtenergie des mindestens einen Strahlungspulses so steuern soll, dass er größer als 0,5 J ist.

6. Vorrichtung nach Anspruch 2, wobei die Steuereinrichtung (104) eine Gesamtenergie des mindestens einen Strahlungspulses so steuern soll, dass er zwischen 0,5 J und 35 J liegt.

7. Vorrichtung nach Anspruch 2, wobei die Steuereinrichtung (104) eine Gesamtenergie des mindestens einen Strahlungspulses so steuern soll, dass er zwischen 1 J und 20 J liegt.

8. Vorrichtung nach Anspruch 2, wobei die Strahlungsquellenanordnung (102) einen Abtragungslaser einschließt.

9. Vorrichtung nach Anspruch 8, wobei der Abtragungslaser ein CO2-Laser und/oder ein Glasfaserlaser ist.

10. Vorrichtung nach Anspruch 9, wobei die Steuereinrichtung (104) eine Pulsenergie, eine Pulsdauer und/oder eine Pulsfrequenz basierend auf einem von der Sensoranordnung (106) empfangenen Signal steuern soll.

11. Vorrichtung nach einem der Ansprüche 1 bis 9, wobei die optische Einrichtung (105) eine Vielzahl von Pulsen auf eine Vielzahl von speziellen Stellen auf einer Oberfläche des Hautgewebes lenken soll.

12. Vorrichtung nach Anspruch 11, wobei die optische Einrichtung (105) einen Abstand zwischen benachbarten der speziellen Stellen so steuern soll, dass er größer als 1 mm ist.

13. Vorrichtung nach Anspruch 11, wobei die optische Einrichtung (105) einen Abstand zwischen benachbarten der speziellen Stellen so steuern soll, dass er größer als 1,5 mm ist.

14. Vorrichtung nach Anspruch 12, des Weiteren umfassend ein Handstück (107), welches mindestens einen Teil der optischen Einrichtung (105) umfasst.

## Revendications

1. Appareil pour faire chauffer des graisses sous-cutanées (130), comprenant :
un agencement (102) formant source de radiation configuré pour fournir au moins une impulsion de radiation ablative ;
un agencement de commande (104) configuré pour commander au moins une propriété de ladite au moins une impulsion de radiation ;
un agencement optique (105) configuré pour diriger ladite au moins une impulsion de radiation vers un tissu cutané ; et
un agencement détecteur (106) prévu en communication avec l'agencement de commande (104),
dans lequel ladite au moins une impulsion de radiation a une énergie supérieure à 0,35 J et un diamètre focal inférieur à 0,5 mm,
dans lequel l'un au moins parmi l'agencement de commande (104) ou l'agencement optique (105) est configuré pour commander et/ou diriger ladite au moins une impulsion de radiation pour faire une ablation d'au moins un trou (150) à travers une couche dermique entière du tissu cutané (120), et
dans lequel l'agencement détecteur (106) est configuré pour détecter une présence de l'une au moins parmi des graisses chauffées émanant dudit au moins un trou pratiqué par ablation (150) ou les graisses vaporisées émanant dudit au moins un trou pratiqué par ablation.

2. Appareil selon la revendication 1, dans lequel l'agencement de commande (104) est adapté pour commander une durée de ladite au moins une impulsion de radiation entre 10 msec et 1 seconde.

3. Appareil selon la revendication 1, dans lequel l'agencement de commande (104) est adapté pour commander une durée de ladite au moins une impulsion de radiation entre 0,25 seconde et 0,5 seconde.

4. Appareil selon la revendication 2, dans lequel l'agencement de commande (104) est adapté pour commander un diamètre focal de ladite au moins une impulsion de radiation à une valeur inférieure à 0,2 mm.

5. Appareil selon la revendication 2, dans lequel l'agencement de commande (104) est adapté pour commander une énergie totale de ladite au moins une impulsion de radiation à une valeur supérieure à 0,5 J.

6. Appareil selon la revendication 2, dans lequel l'agencement de commande (104) est adapté pour commander une énergie totale de ladite au moins une impulsion de radiation à une valeur entre 0,5 J et 35 J.

7. Appareil selon la revendication 2, dans lequel l'agencement de commande (104) est adapté pour commander une énergie totale de ladite au moins une impulsion de radiation à une valeur entre 1 J et 20 J.

8. Appareil selon la revendication 2, dans lequel l'agencement formant source de radiation (102) comprend un laser d'ablation.

9. Appareil selon la revendication 8, dans lequel le laser d'ablation est au moins un parmi un laser à CO₂ et un laser à fibre.

10. Appareil selon la revendication 9, dans lequel l'agencement de commande (104) est adapté pour commander un paramètre au moins parmi une énergie d'impulsion, une durée d'impulsion, ou une fréquence d'impulsion, sur la base d'un signal reçu depuis l'agencement détecteur (106).

11. Appareil selon l'une quelconque des revendications 1 à 9, dans lequel l'agencement optique (100) est adapté à diriger une pluralité d'impulsions vers une pluralité d'emplacements particuliers sur une surface du tissu cutané.

12. Appareil selon la revendication 11, dans lequel l'agencement optique (100) est adapté pour commander une distance entre des emplacements adjacents parmi les emplacements particuliers à une valeur supérieure à 1 mm.

13. Appareil selon la revendication 11, dans lequel l'agencement optique (100) est adapté pour commander une distance entre des em placements adjacents parmi les emplacements particuliers à une valeur supérieure à 1,5 mm.

14. Appareil selon la revendication 12, comprenant en outre une pièce à main (107) qui inclut au moins une portion de l'agencement optique (100).
